# EUROPEAN PATENT SPECIFICATION

(11) **EP 3 964 116 B1**
(45) Date of publication and mention of the grant of the patent: **28.10.2026**
(21) Application number: 20194137.4
(22) Date of filing: 02.09.2020
(51) Int. Cl.: A61B 1/00, A61B 1/05, A61B 1/06, A61B 1/07

(54) **ENDOSCOPE TIP PART AND METHOD OF ASSEMBLING AN ENDOSCOPE TIP PART**
ENDOSKOPSPITZENTEIL UND VERFAHREN ZUM ZUSAMMENBAU EINES ENDOSKOPSPITZENTEILS
PARTIE DE POINTE D'ENDOSCOPE ET MÉTHODE D'ASSEMBLAGE DE LA PARTIE DE POINTE D'ENDOSCOPE

(43) Date of publication of application: 09.03.2022
(73) Proprietor: Ambu A/S, 2750 Ballerup (DK)
(72) Inventor: JOCHUMSEN, Hans, 3450 Allerød (DK); SØRENSEN, Morten, 2750 Ballerup (DK); JENSEN, Thomas, Bachgaard, 1721 Copenhagen V (DK); JACOBSEN, Morten, 2970 Hørsholm (DK)
(74) Representative: COPA Copenhagen Patents

(56) References cited:
- JP-A- 2005 304 812
- JP-A- 2009 125 528
- US-A1- 2019 175 007
- US-A1- 2019 282 077

## Description

### TECHNICAL FIELD

The present disclosure relates to an endoscope tip part, a light guide component for an endoscope tip part, an endoscope, and a method of assembling an endoscope tip part.

### BACKGROUND

Insertion endoscopes are well-known devices in the medical field for visually examining the interior of a hollow organ or cavity of a body, such as lungs, by means of inserting an insertion portion of the endoscope. The insertion portion of the endoscope comprises an elongated insertion tube, a tip part, and a bending section connecting the insertion tube with the tip part. The endoscope typically has a handle connected to the insertion tube and positioned at the proximal end of the endoscope as seen from the operator. The endoscope further has an imaging subassembly with an image sensor built in the tip part at the distal end of the endoscope.

This definition of proximal as being closest to an operator and distal as being furthest from an operator is used throughout this disclosure. Illumination of the area in front of the distal tip of the endoscope is normally required, in particular the field of vision of the image sensor. One known way of achieving such illumination is to incorporate one or more light sources, such as Light-emitting Diodes (LEDs), and one or more light guides in the tip part of the endoscope, e.g. as mentioned in EP 19 161 893 disclosing a disposable endoscope.

The present disclosure makes a distinction between optical fibres and light guides. Optical fibres in this context are to be understood as highly elongated and flexible elements, where the length is several orders of magnitude larger than the diameter, providing the fibres with a high degree for flexibility to allow them to conduct luminous flux to a desired place. Light guides are to be understood as much shorter, preferably rigid elements adapted to guide and distribute light in a desired manner. Furthermore, while optical fibres are used to transport light, as used herein light guides are primarily used to reshape the incoming light beam.

The bending section is provided in order to manoeuvre the endoscope inside the body cavity. The bending section has increased flexibility, e.g. achieved by a number of articulated segments of which the tip part forms the distalmost segment. Bending or straightening of the bending section in the insertion part of the endoscope is typically done by tensioning or slacking, respectively, steering wires running from the tip part through the remainder of articulated segments and along the inside of the elongated insertion tube to a control mechanism, such as a control lever, of the handle.

Data and/or power cables for the image sensor and other electronics, such as LED lighting accommodated in the tip part at the distal end of the endoscope, run along the inside of the elongated insertion tube and the bending section from the handle to the tip part. Furthermore, a working channel may run along the inside of the insertion tube and the bending section from the handle to the tip part, e.g. allowing liquid to be removed from the body cavity or allowing the insertion of medical tools or surgical instruments into the body cavity.

One of the parameters driving the number of medical applications for which the endoscope can be used, is the outer diameter of the insertion tube and the endoscope tip part. Thus, an ongoing goal in the field is to minimise this outer diameter to enable the endoscope to inspect smaller bodily ducts, e.g. further into the branches of the bronchial tree or nasal passages. However, in such a miniaturised tip part it is difficult to ensure consistent optical performance of the image sensor and light source.

Prior art document JP 2009 125528 A teaches an insertion endoscope according to the preamble of independent claim 1.

### SUMMARY

In light of the above, it may be seen as an object of the present disclosure to provide a miniaturised endoscope tip part while reducing the variance in the optical properties of the imaging means and light source.

Another object of the present disclosure is to provide a method of assembling a tip part which reduces the variance in the optical properties of the imaging means and light source.

One or more of these objects may be met by aspects of the present disclosure as described in the following.

A first aspect of this disclosure according to the invention, relates to an endoscope tip part for an endoscope for visually inspecting inaccessible places, such as human body cavities, the endoscope tip part having an exterior surface and extending along a longitudinal axis and comprising:
- a tip housing extending along the longitudinal axis and at least partially enclosing a sealed interior space, the tip housing including:
   ∘ an exterior housing surface for forming a first part of the exterior surface of the endoscope tip part, and
   ∘ a transparent portion, such as a window or a front lens, having an interior surface facing the sealed interior space of the endoscope tip part and an exterior surface forming a second part of the exterior surface of the endoscope tip part;
- an imaging subassembly including an image sensor viewing in an optical direction through the transparent portion of the tip housing;
- a light guide component comprising a first and a second light guide being spaced apart by a gap and extending along a respective longitudinal centre line, wherein each light guide has a proximal end, a distal end, a light entry surface at the proximal end, a light exit surface at the distal end, and a circumferential surface extending from the light entry surface to the light exit surface around the respective longitudinal centre line, and a crossmember extending transversely from the first light guide to the second light guide so as to form a unitary rigid light guide component, wherein the light guides are configured for propagating light received through the respective light entry surface out through the respective light exit surface and the transparent portion of the tip housing;
- a first light source having a light-emitting surface and being configured to emit light, from the light-emitting surface, in a central illumination direction to be received by the light entry surface of the first light guide, preferably the central illumination direction being at least partially oriented in the optical direction of the image sensor; and
- an electrical circuit comprising a first circuit portion in electrical communication with the image sensor and preferably a second circuit portion in electrical communication with the first light source, the electrical circuit being configured for transmitting an image signal generated by the image sensor indicative of the view in the optical direction, preferably to a handle of the endoscope;
wherein the first light source, the image sensor, and the electrical circuit are accommodated in the sealed interior space of the tip housing, wherein the first light source is attached, preferably directly attached, to the light guide component and preferably positioned proximally relative to the image sensor.

By attaching the light source, preferably directly, to the light guide component, the loss of and variation in power of light due to varying offset position of the light source in relation to the light guide component. Furthermore, the variance in a gap distance between the light source and the light guide component can be reduced or even avoided by directly attaching the light source to the light guide component. This has the advantage of reducing power losses and power variances. A further advantage of such an arrangement is reducing the risk of adhesive sealing the interior space enters between the light source and the light guide component during application as this can cause reduced optical performance, such as reduced illumination power or illumination colour shifting. An additional advantage is that such an arrangement is easier to assemble, especially when the tip part is miniaturised as the light source and the light guide component is an integrated assembly.

In the present disclosure, a direct attachment between two parts may be defined as the two parts are attached via attachment means without an intermediate connector part. An example of a direct attachment are two parts adhered together so that the same adhesive contacts both elements. An example of an indirect attachment is the attachment between the tip part and the handle which is provided via the bending section and the insertion tube.

Additionally or alternatively, the light guide(s) may be configured to transmit light received by the respective light entry surface, past the imaging subassembly, and out through the respective light exit surface.

Additionally or alternatively, the first and/or second light guide(s) consist(s) essentially of a polymer material and the circumferential surface of the first and/or second light guide defines a polymer-air interface.

Additionally or alternatively, the endoscope may comprise one or more optical redirection component(s) arranged between the corresponding light source and the corresponding light guide. The optical component(s) may be configured for reflecting or mirroring light emitted from the light source(s) towards the light entry surface of the corresponding light guide.

Additionally or alternatively, the tip housing may extend along a longitudinal axis and may comprise a wall extending circumferentially around the longitudinal axis. The wall may have a cylinder shell shape. The transparent portion may close off a distal end of the wall.

Additionally or alternatively, the tip housing may be formed as a monolithic piece. The transparent portion of the tip housing is preferably formed of a transparent material.

Additionally or alternatively, the tip housing may be manufactured by a single-shot injection moulding, preferably of a transparent material. This is a simple and cheap way of manufacturing the tip housing. Alternatively, the tip housing may be manufactured by a multi-shot injection moulding process comprising at least two shots. The wall may be formed by a shot, preferably of an opaque material, and the transparent portion may be formed by another shot, preferably of a transparent material. By forming part of the tip housing in a different material allows tailoring that part to have specific properties, for instance that part may be formed of an opaque material which may prevent stray light from entering the image sensor.

Additionally or alternatively, the transparent portion is preferably a window. The transparent portion may be formed in a distal end face of the tip housing.

Additionally or alternatively, the endoscope tip part may comprise a plug sealing a proximal opening of the wall, e.g. by an adhesive. The plug may be formed separately from housing.

Additionally or alternatively, the sealed interior space is filled with air. In particular, the light guide(s) or the circumferential surface of the light guide(s) being surrounded by air. The interior space may be accessible by the proximal opening, and the tip housing may enclose the interior space so that the proximal opening is the only access to the interior space.

Additionally or alternatively, the light source(s) may comprise a light-emitting diode configured for emitting substantially white light. The light-emitting diode may be a single light-emitting diode, which is also known as a single-die light-emitting diode. Additionally, the light-emitting diode may be phosphor-based which is particularly suited, as it can emit white light and is compact.

In any case, the light-emitting diode may comprise a semiconductor die surrounded by a cover which is typically epoxy-based. Alternatively, the light source(s) may comprise a plurality of light-emitting diodes, such as three, each configured for emitting a light of a different wavelength, such as red, green, and blue light. The plurality of light-emitting diodes may be covered by a cover and may be arranged so that the light emitted from the cover is substantially white. In both cases, an exterior surface of the cover may define the light-emitting surface of the light source(s). Alternatively, the light source(s) may comprise one or more optical fibres configured for transmitting light from an external light source, such as a light-emitting diode, positioned outside of the endoscope tip part, such as positioned in the handle. The light-emitting surface(s) may thus form part of the one or more optical fibres.

Additionally or alternatively, the central illumination direction of the light source(s) may be at least partially oriented in, preferably parallel to, the optical direction of the image sensor. This may have the advantage of reducing light losses, as the redirection of the emitted light beam of the light source is reduced. The central illumination direction of the light source(s) may intersect the light entry surface of the corresponding light guide.

Alternatively, the central illumination direction of the light source(s) may be substantially perpendicular to the optical direction of the image sensor. This has the advantage of allowing for a more compact arrangement of the light source(s).

Additionally or alternatively, the imaging subassembly may comprise a lens arrangement including one or more lenses aligned with the optical direction and between the interior surface of the transparent portion and the image sensor so that the image sensor views through the lens arrangement and the transparent portion.

Additionally or alternatively, the first light source may be an electrical component, such as one or more light-emitting diodes, and the electrical circuit may comprise a second circuit portion in electrical communication with the first light source.

Additionally or alternatively, the electrical circuit may be provided on a printed circuit board (PCB). The electrical circuit may comprise a main circuit portion for interconnecting various electronic components, e.g. capacitors, transistors, and the like, of the printed circuit board. The first circuit portion of the electrical circuit may electrically connect the image sensor to the main circuit portion and the second circuit portion of the electrical circuit may electrically connect the light source(s) with the main circuit portion.

The first circuit portion and/or the second circuit portion may be flexible so that, prior to assembly, the image sensor and light source(s) are movable relative to each other. In this case, the electrical circuit may be provided on a flexible circuit board (FPC). This may be an advantage in assembly, as the image sensor and light source(s) can be moved relatively to their desired position irrespective of production tolerances.

The electrical circuit may be configured for transmitting the image signal to an electrical component, such as a circuit board, positioned in the handle. The image signal may be transmitted from the electrical circuit of the endoscope tip part via a wired connection or a wireless connection. Thus, the electrical circuit does not necessarily comprise a cable extending between the tip part and the handle for transmitting the image signal.

Additionally or alternatively, the endoscope tip part may comprise a frame part for supporting the electrical circuit. In particular, the electrical circuit may be provided on a folded FPC being folded around the frame part.

According to the invention, the light guide component comprises a second light guide having a light entry surface and a light exit surface, the second light guide being configured for propagating light received through the light entry surface out through the light exit surface and the transparent portion of the tip housing. Additionally, the endoscope tip part comprises a second light source having a light-emitting surface and being configured to emit light from the light-emitting surface in a central illumination direction to the light entry surface of the second light guide, preferably the central illumination direction of the second light source being at least partially oriented in the optical direction of the image sensor. Additionally or alternatively, a surface normal of the light-emitting surface of the second light source may be at least partially oriented in the optical direction of the image sensor.

The second light source may be attached, preferably directly attached, to the light guide component and preferably positioned proximally relative to the image sensor, and wherein the first and second light guides extend side-by-side or in parallel on opposite sides of the imaging subassembly, preferably the image sensor.

By having a light guide component with both a first and a second light guide, assembly of the tip part is further improved as the light guides can be handled as a single component. Further advantages of attaching the first light source to the light guide component as previously described is achieved also for the second light source.

Additionally or alternatively, the light guide exit(s) may comprise a collar at least partially surrounding the respective light guide, and the interior surface of the transparent portion may comprise a seat or seats for receiving the respective collar. Each seat may be formed as a rail or a depression for retaining the respective light guide. This may be advantageous for assembling the endoscope tip part.

Additionally or alternatively, the light guide component may be formed separately from the tip housing, and wherein the first light guide exit and optionally the second light guide exit are oriented towards the interior surface of the transparent portion.

Such an arrangement is particularly advantageous for assembly, as the light source(s) can be attached to the light guide component outside of the tip housing and then subsequently assembled with the tip housing.

The second light guide may be identical or be symmetric to the first light guide, and the second light source may be identical or be symmetric to the first light source. This may improve the light distribution in the field of view of image sensor.

Additionally or alternatively, the tip housing may comprise a distal end face and a proximal opening providing access to the sealed interior space, wherein the distal end face is preferably positioned opposite of the proximal opening, and wherein the distal end face comprises at least part of the exterior surface of the transparent portion, preferably the entirety of the exterior surface of the transparent portion.

Additionally or alternatively, the first and/or the second light source(s) may be at least partially overlapping the imaging subassembly, preferably the image sensor, when viewed in a cross-section perpendicular to the longitudinal axis.

This has the advantage of allowing a more compact arrangement of the light source(s) and imaging subassembly, as the light source(s) can be packed behind the image sensor and thus reduce(s) the combined diameter of the light source(s), and the imaging subassembly thereby allowing a smaller outer diameter of the tip housing.

Additionally or alternatively, the first and/or the second circuit portion(s) may be flexible circuit structures, preferably so that, prior to assembly, the image sensor and light source(s) are movable relative to each other via flexing of first and/or second circuit portions.

Since optimum optical performance is achieved by positioning the imaging subassembly and light source(s) with a predetermined relative distance, the flexible circuit portion(s) has/have the advantage of allowing the assembler to move the parts relative to each other and thus absorb this variance.

Additionally or alternatively, the first and/or the second light guide(s) may consist essentially of a transparent material with a first refractive index, and wherein the light-emitting surface of the light source(s) is adhered to the light entry surface of the respective light guide by an adhesive preferably with a second refractive index within +/- 0.2, preferably within +/- 0.1, of the first refractive index.

By adhering the light source(s) to the light entry surface of the respective light guide, the gap between these two elements is eliminated, thus increasing the illumination power of the light source(s). By configuring the first and second refractive indices to be similar, the less desirable optical effects of having an adhesive layer between the light guide and light source is reduced or even eliminated.

A second aspect of this disclosure, not according to the invention, relates to a light guide component for an endoscope tip part, such as an endoscope tip part according to the first aspect, the light guide component comprising:
- a first and a second light guide being spaced apart by a gap and extending, preferably side-by-side or in parallel, along a respective longitudinal centre line, wherein each light guide has a proximal end, a distal end, a light entry surface at the proximal end, a light exit surface at the distal end, and a circumferential surface extending from the light entry surface to the light exit surface around the respective longitudinal centre line, and
- a crossmember extending transversely from the first light guide to the second light guide, preferably over the gap so as to form a unitary rigid light guide component,
wherein the light guides are configured for propagating light received through the respective light entry surface out through the respective light exit surface, preferably while minimising light loss through the respective circumferential surface.

Such a light guide component has the advantage of easing assembly of two spaced-apart light guides in a miniaturised tip housing since they can be handled as a unitary component by means of the crossmember.

Additionally or alternatively, the light guides may be reflection-symmetric in a first plane extending at a distance between the light guides, i.e. does not intersect the light guides.

Additionally or alternatively, the first and/or the second light guide(s) may be reflection-symmetric in a second plane comprising the longitudinal centre line and preferably intersecting the first plane. Additionally or alternatively, the first and/or the second light guide(s) may be reflection-asymmetric in a third plane being perpendicular to the second plane and comprising the longitudinal centre line and preferably intersecting the first plane.

Additionally or alternatively, the longitudinal centre line of each light guide may be straight or curved. The longitudinal centre lines may be extending side-by-side or preferably in parallel.

Additionally or alternatively, each light guide may be a single moulded component.

Additionally or alternatively, each light guide may have a varying cross-section along the respective longitudinal centre line. Each light guide may comprise a transition portion including the respective light entry surface having a varying cross section along the longitudinal centre line. Each light guide may further comprise a straight portion extending directly from the transition portion, the straight portion having a substantially constant cross-section along the longitudinal centre line. The transition portion may taper with a taper angle with respect to the longitudinal axis, wherein the taper angle is in the range of 1 - 12 degrees, preferably in the range of 5 - 10 degrees, or more preferably in the range of 7 - 10 degrees.

Additionally or alternatively, the light guide(s), in particular the straight portion of each light guide, may have a width and a height. The width may be in the range of 0.35 - 0.55 mm or preferably in the range of 0.4 - 0.5 mm. The height may be in the range of 0.6 - 0.8 mm or preferably in the range of 0.65 - 0.75 mm.

Additionally or alternatively, the gap spacing apart the light guides may have a distance transversely to the longitudinal axis in the range of 1.3 - 1.8 mm, preferably in the range of 1.4 - 1.7 mm.

Additionally or alternatively, the first and/or second light guide(s) may be made of a material with a refractive index that is higher than the refractive index of air. This ensures that the critical angle for total internal reflection of light incident on the circumferential surface of the respective light guide is also increased, thus increasing the ability of the respective light guide to transmit light from entry to exit.

Additionally or alternatively, the first light guide component comprises a rail or rails at least partially surrounding one or both light entry surfaces, the rail(s) being configured to retain a light source attached to the respective light guide.

Additionally or alternatively, the crossmember may be opaque or may be formed of an opaque material.

In the context of this disclosure, a light guide is not regarded as the same as an optical fibre. An optical fibre is typically long and thin, i.e. the length is several orders of magnitudes larger than the diameter and thus flexible providing the fibres with a high degree of flexibility to allow them to transmit luminous flux to a desired place without reshaping the light beam. Optical fibres are also typically arranged in a bundle. In contrast, a light guide is to be understood as much shorter, i.e. typically the length is typically within the same or one order of magnitude larger than the diameter, and preferably rigid, typically made in a single piece (e.g. monolithically formed) of a transparent material. A light guide also differs in function in that it is adapted to guide and reshape the incoming light beam in a desired manner. Further an optical fibre typically comprises an opaque sheath while a light guide typically is monolithically formed without a sheath.

Additionally or alternatively, the crossmember may extends transversely, preferably over the gap, from the proximal end of the first light guide to the proximal end of the second light guide.

This may be advantageous for facilitating assembly, as the assembler can push on the crossmember to ensure that the light guide component is desirably positioned in the housing. The crossmember may also in this way partition the tip housing in a proximal and distal portion and may thus aid in sealing the light guide component.

Alternatively, the crossmember may extend transversely over the gap from the distal end of the first light guide to the distal end of the second light guide. Alternatively, the crossmember may extend transversely over the gap from a central portion of the first light guide to a central portion of the second light guide, the central portions being located between the proximal and distal portion of the respective light guide.

Additionally or alternatively, the crossmember may partition the interior space in a proximal interior space and a distal interior space closed by the crossmember, the distal interior space accommodating the imaging subassembly.

Additionally or alternatively, the light guide component is formed monolithically, and may preferably be manufactured by a moulding process, such as an injection moulding process.

Additionally or alternatively, the light guide component, i.e. the first light guide, second light guide, and the crossmember, is made of a single transparent polymer material.

Additionally or alternatively, the light guide component may be manufactured by single-shot injection moulding, preferably of a transparent material. This is a simple way of manufacturing the light guide component. Alternatively, the light guide component may be manufactured by a multi-shot injection moulding process comprising at least two shots. The crossmember may be formed by a shot, preferably of an opaque material, and the first and second light guides may be formed by another shot, preferably of a transparent material. By forming part of the crossmember in an opaque material may prevent stray light from entering the image sensor.

Additionally or alternatively, the light entry surfaces of the first and second light guides may be planar and preferably parallel, and/or light exit surfaces of the first and second light guides may be planar and preferably parallel. In a preferably embodiment, all the light entry surfaces and the light exit surfaces are planar and parallel.

Additionally or alternatively, a gap may be located between the crossmember and the tip housing. The gap may be substantially uniform about the longitudinal axis. The gap may be sealed by an adhesive.

A third aspect of this disclosure relates to an endoscope for visually inspecting inaccessible places such as human body cavities, the endoscope comprising:
- a handle for gripping by an operator and comprising a control device;
- an endoscope tip part according to the first aspect of this disclosure;
- an insertion tube for insertion into a patient, the insertion tube extending from the handle to the endoscope tip part and comprising a bending section;
- **one or more** cables running through the insertion tube and electrically connecting the electrical circuit of the endoscope tip part with the handle; and
- at least one steering wire connecting the handle with a distal end of the bending section so that manipulation of the control device causes bending of the bending section.

A fourth aspect of this disclosure relates to an endoscope system for visually inspecting inaccessible places, such as human body cavities, the endoscope system comprising a monitor and an endoscope according to the third aspect or an endoscope comprising an endoscope tip part according the first aspect, wherein the endoscope is connectable to the monitor, and the monitor is configured for displaying an image captured by the image sensor of the endoscope tip part.

A fifth aspect of this disclosure relates to a method for assembling an endoscope tip part, such as an endoscope tip part according to the first aspect, the endoscope tip part extending along a longitudinal axis, the method comprising the steps of:
- providing:
   ∘ a tip housing extending along the longitudinal axis and at least partially enclosing an interior space, the tip housing including:
      ∘ a proximal opening providing access to the interior space,
      ∘ an exterior housing surface for forming an exterior surface of the endoscope tip part, and
      ∘ a transparent portion, such as a window or a front lens, having an interior surface and an exterior surface, the interior surface facing the interior space of the endoscope tip part and the exterior surface facing the exterior of the endoscope tip part,
      ∘ an imaging subassembly including an image sensor viewing in an optical direction,
      ∘ a light guide component comprising a first and a second light guide being spaced apart by a gap and extending along a respective longitudinal centre line, wherein each light guide has a proximal end, a distal end, a light entry surface at the proximal end, a light exit surface at the distal end, and a circumferential surface extending from the light entry surface to the light exit surface around the respective longitudinal centre line, and a crossmember extending transversely from the first light guide to the second light guide so as to form a unitary rigid light guide component, wherein the light guides are configured for propagating light received through the respective light entry surface out through the respective light exit surface and the transparent portion of the tip housing,
      ∘ a first light source having a light-emitting surface and being configured to emit light from the light-emitting surface in a central illumination direction, and a second light source having a light-emitting surface and being configured to emit light from the light-emitting surface in a central illumination direction, and
      ∘ an electrical circuit comprising a first circuit portion in electrical communication with the image sensor and a second circuit portion in electrical communication with the light source(s), the electrical circuit being configured for transmitting an image signal generated by image sensor indicative of the view in the optical direction, preferably to a handle of the endoscope, wherein at least one of the first and second circuit portions is flexible so the image sensor and light source(s) are movable relative to each other;
- adhering the first and second light sources, preferably directly, to the light guide component, preferably to the first light guide, so that light emitted by the first light source is received by the light entry surface of the first light guide, and so that light emitted by the second light source is received by the light entry surface of the second light guide, and so that the imaging subassembly, the light guide component, and the electrical circuit forms a main subassembly;
- inserting the main subassembly through the proximal opening of the tip housing into the interior space so that the optical direction of the image sensor extends through the transparent portion, and so that surface normals of the light exit surface of the light guides are at least partially oriented towards the interior surface of the transparent portion;
- fixing the main subassembly to the tip housing and sealing the proximal opening to seal the interior space.

The light guide component is provided according to the second aspect of this disclosure.

Additionally or alternatively, the method may further comprise a step, preferably performed prior to the step of inserting, of:
- arranging the main subassembly in a jig, adjusting the light guide component relative to the imaging subassembly to a predetermined relative position along the longitudinal direction via flexing of the first and/or second circuit portion(s), and subsequently fixing, preferably by adhering, the light guide component relative to the imaging subassembly,
wherein the step of inserting the main subassembly comprises inserting the main subassembly along the longitudinal direction until a distal end of the imaging subassembly abuts an interior distal portion of the tip housing, preferably the interior surface of the transparent portion, and until a distal end of the light guide component, preferably the light exit surface of the first light guide, abuts an interior distal portion of the tip housing, preferably the interior surface of the transparent portion.

In the present disclosure, a partial orientation of a surface in a direction may be defined as an angle between a surface normal of said surface and said direction being acute or zero, e.g. that the light-emitting surface is at least partially oriented in the optical direction may be defined as a surface normal of the light-emitting surface and the optical direction form an acute angle or are parallel, and an orientation of a surface towards an element may be defined as a surface normal of said surface intersects said element.

A person skilled in the art will appreciate that any one or more of the above aspects of this disclosure and embodiments thereof may be combined with any one or more of the other aspects of this disclosure and embodiments thereof.

### BRIEF DESCRIPTION OF THE DRAWINGS

Embodiments of this disclosure will be described in more detail in the following with regard to the accompanying figures. The figures show one way of implementing the present invention and are not to be construed as being limiting to other possible embodiments falling within the scope of the attached claim set.
Fig. 1 is a schematic perspective illustration of an endoscope according to this disclosure,
Fig. 2 is a schematic perspective illustration of a distal tip and a bending section of the endoscope shown in Fig. 1,
Fig. 3 is a schematic perspective illustration of the distal tip of Fig. 2 without the bending section,
Fig. 4a is a schematic perspective cross-sectional illustration of the distal tip along the A-A line of Fig. 3,
Fig. 4b is a schematic side cross-sectional illustration of the distal tip along the A-A line of Fig. 3 and illustrates detail view A,
Fig. 5 is a schematic side cross-sectional illustration of the distal tip along the B-B line of Fig. 3,
Figs. 6a-6b are schematic illustrations of the distal tip of Fig. 3 without the tip housing in a perspective and side view, respectively,
Figs. 7a-7b are schematic illustrations of a second embodiment of a distal tip without the tip housing in a distal and proximal perspective view, respectively,
Fig. 8 is a schematic top view of a third embodiment of a distal tip without the tip housing, and
Fig. 9 is a schematic perspective illustration of a monitor connectable to the endoscope of Fig. 1.
Fig. 10a is a schematic perspective illustration of the light guide component alone.
Fig. 10b is a schematic cross-sectional illustration of the light guide component in a plane perpendicular to the longitudinal axis of the distal tip part.
Fig. 11 is an enlarged view of detail view A as shown in Fig. 4b and illustrates transition portion of the light guide defining the taper angle a.

### DETAILED DESCRIPTION

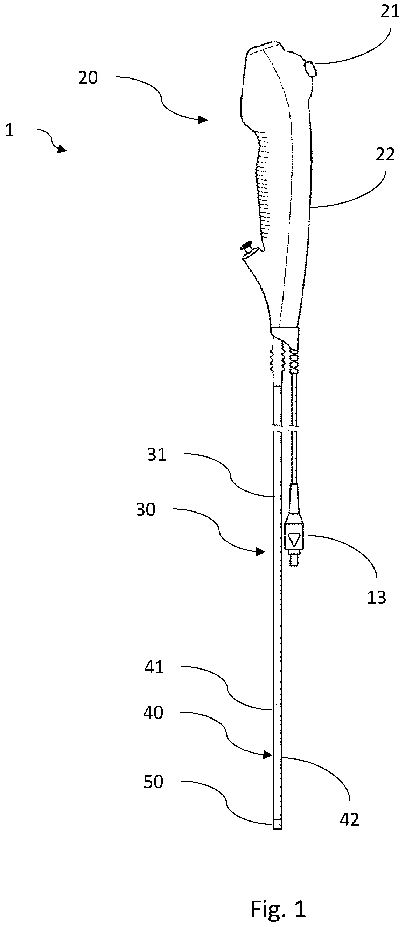

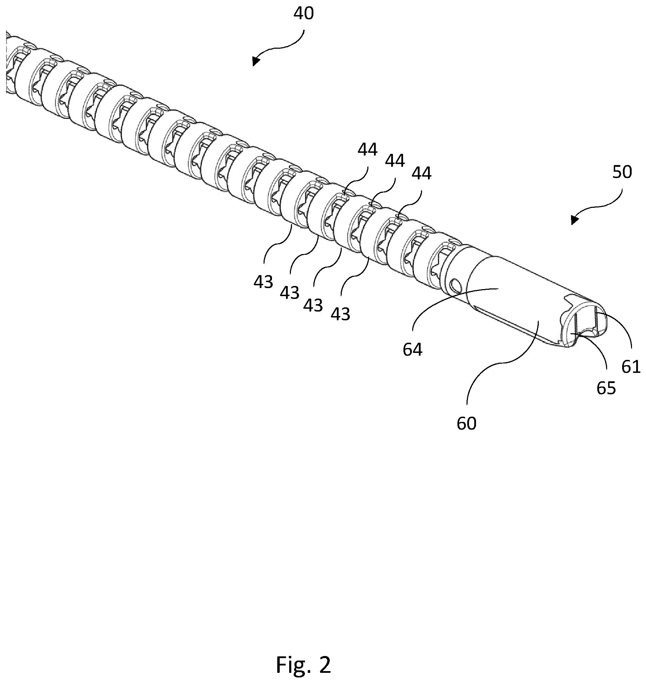

Fig. 1 illustrates an endoscope 1, which is disposable and not intended to be cleaned and reused. The endoscope 1 comprises an endoscope tip part 50, a handle 20 with a handle housing 22 for gripping and a control lever 21, and an insertion tube 30 for insertion into a patient and extending between the handle 20 and a proximal end 41 of a bending section 40. The insertion tube 30 has an exterior tubular surface 31 providing a barrier against the surroundings of the endoscope 1. The bending section 40 comprises articulated segments 43, hinges 44 connecting the segments 43 as best seen in Fig. 2, and a thin outer sleeve 42. The endoscope 1 further comprises two steering wires (not shown) extending inside the insertion tube 30 from the control lever 21 of the handle 20 to the distal tip 50 and arranged in a Bowden cable configuration so that an operator can, upon manipulation of the control lever 21, articulate the segments 43 of the bending sections about their respective hinges 44 to cause bending of the bending section 40. The thin outer sleeve 42 covers the segments 43, the hinges 44, and the gaps between the segments 43d, thereby providing a smooth outer surface for the bending section 40 in order to improve the comfort of a patient undergoing endoscopy, especially when the operator articulates the bending section 40. The thin outer sleeve 42 further provides an additional layer of sealing for the connection between the endoscope tip part 50 and the bending section 40. Furthermore, a working channel (not shown) run along the inside of the insertion tube from the handle 20 to a working channel opening (not shown) of the endoscope tip part 50, e.g. allowing liquid to be removed from the body cavity or allowing the insertion of medical tools or surgical instruments into the body cavity.

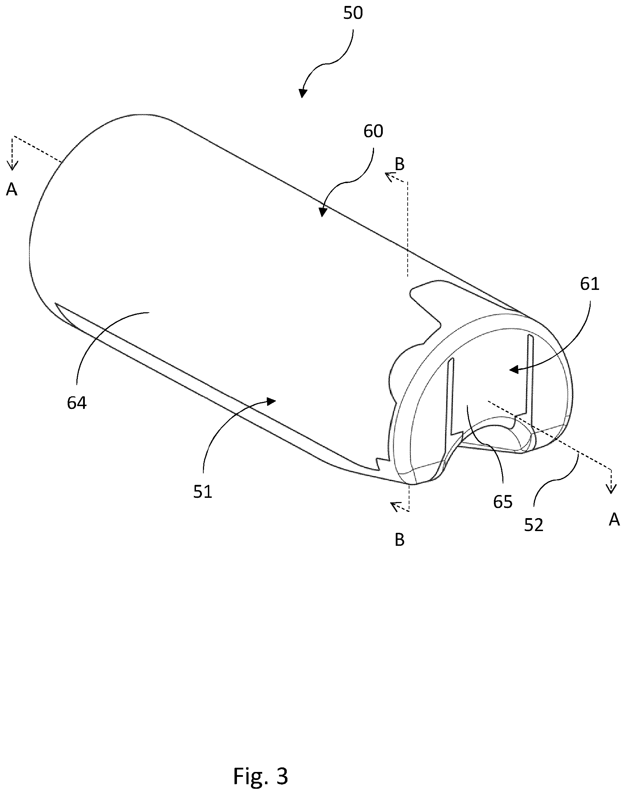

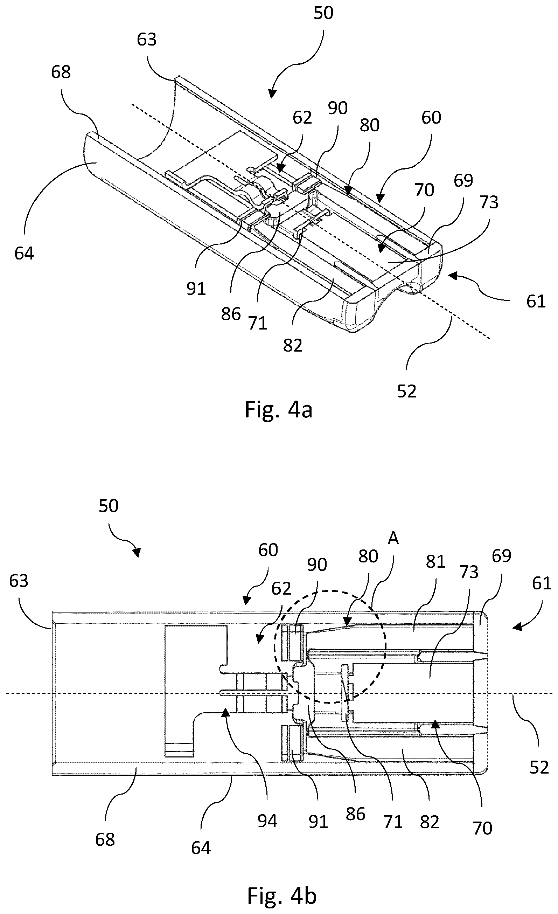

Turning to Figs. 3 and 4a-4b, the endoscope tip part 50 can be seen in greater detail without the bending section 40. The tip part 50 comprises an exterior surface 51 facing the exterior of the tip part 50, and a tip housing 60 comprising a cylindrical-shell shaped circumferential wall 68 extending circumferentially around a longitudinal axis 52 and surrounding an interior space 62 as best seen in Figs. 4a-4b. The circumferential wall 68 is closed at a distal end of the tip housing 60 by a distal end wall 69 and has a proximal opening 63, which provides the only access to the interior space 62, at the opposite end of the tip housing 60. The tip housing 60 comprises an opaque portion and a transparent portion including a window 65 arranged in the distal end wall 69. The window 65 has an interior surface 66 facing the sealed interior space 62 and an exterior window surface 67 forming part of the exterior surface 51 of the tip housing 60. The tip housing is manufactured by two-shot injection moulding processes so that the transparent portion 65 is formed by a transparent polymer material shot of the two shot injection moulding process and the opaque portion is formed by an opaque polymer material shot of the two shot injection moulding process so that the tip housing 60 is formed as a monolithic polymer piece. The tip part 50 further comprises a separately formed plug (not shown) arranged in and sealing the proximal opening 63 via an adhesive, thereby fully liquid- and gas-sealing the air-filled interior space 62.

As best seen in Figs. 4a-4b, the tip part 50 comprises an imaging subassembly 70, a first embodiment of a light guide component 80, a first and a second light source 90, 91, and an electrical circuit 94 mounted on a frame part (not shown).

The imaging subassembly 70 includes a lens barrel 73 and an image sensor 71 viewing in an optical direction 72 through the lens barrel 73 and out through the window 65 of the tip housing 60. The lens barrel 73 comprises several lenses enclosed in a barrel. The lenses optimise the view of the image sensor 71 and the barrel forms an optical barrier preventing stray light from entering the image sensor 71.

The first embodiment of the light guide component 80 includes a first and a second light guide 81, 82 spaced apart by a gap and extending in parallel along a respective straight longitudinal central centre line. Each light guide has a proximal end, a distal end opposite of the proximal end, a light entry surface 83 at the proximal end, a light exit surface 84 at the distal end, and a circumferential surface 85 extending from the light entry surface 83 to the light exit surface 84 around the respective longitudinal central centre line. The light entry surfaces 83 of the first and second light guides 80, 81 are planar and parallel, and the light exit surfaces 84 of the first and second light guides 80, 81 are planar and parallel. The circumferential surface 85 defines a polymer-air interface between the light guide component 80 and the interior space 62. The light guide component 80 further includes a crossmember 86 extending transversely from the proximal end of the first light guide 81 to the proximal end of the second light guide 82 over the gap between the light guides 81, 82. The light guide component 80 is monolithically formed of a rigid and transparent polymer material with a refractive index that is higher than the refractive index of air. This ensures that the critical angle for total internal reflection of light incident on the circumferential surface 85 of each light guide 81, 82 is also increased and thus increases the ability of the light guides 81, 82 to propagate light from the respective light entry surface 83 to the respective light exit surface 84 and thereby minimising light loss through the circumferential surfaces 85.

The tip part 50 further comprises a first and a second light source 90, 91 each formed of a single phosphor-based light-emitting diode for emitting substantially white light. Each light source 90, 91 comprises a single semiconductor die (not shown) for emitting light surrounded by an epoxy-based cover with an exterior surface forming a light-emitting surface 92. The light-emitting surface 92 of each light source 90, 91 is adhered to the light entry surface 83 of the respective light guide 80, 81 by an adhesive so that light emitted by each light source 90, 91 is propagated by the respective light guide 80, 81 out through the window 65 to provide illumination for the image sensor 71. Alternatively, the light source could be the distal end of an optical fibre transporting light from an LED arranged in the handle of the endoscope.

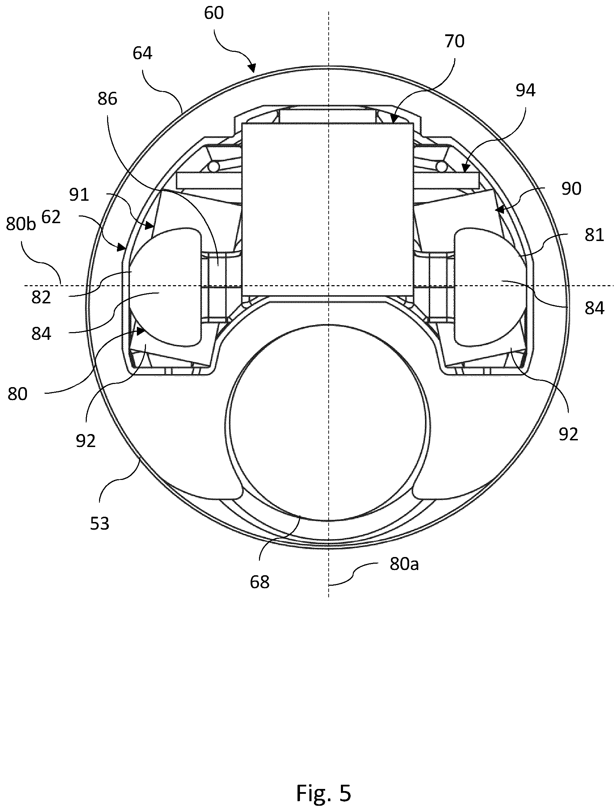

As best seen in Fig. 5, in their intended position the first and the second light sources 90, 91 are partially overlapping the imaging subassembly 70, in particular the lens barrel 73, when viewed in a cross-section normal to the longitudinal axis 52. When accounting for manufacturing and assembly variance, the overlap of the light sources 90, 91 with the imaging subassembly 70 is significant in a large proportion of tip parts.

The light guides 81, 82 have mutual reflection symmetry in a first plane 80a extending equidistantly between the light guides 81, 82 and does not intersect the light guides 81, 82. The first plane 80a comprises the longitudinal direction 52 of the tip part 50. Each light guide 81, 82 has individual reflection symmetry in a second plane 80b comprising the longitudinal central centre line of each light guide 81, 82 and being perpendicular to the first plane 80a. Each light guide is asymmetric in a respective third plane 89. The third plane 89 is perpendicular to the second plane 80b and parallel to the first plane 80a and comprises the longitudinal central centre line of the respective light guide 81, 82. This allows more compact packing of the elements of the tip housing.

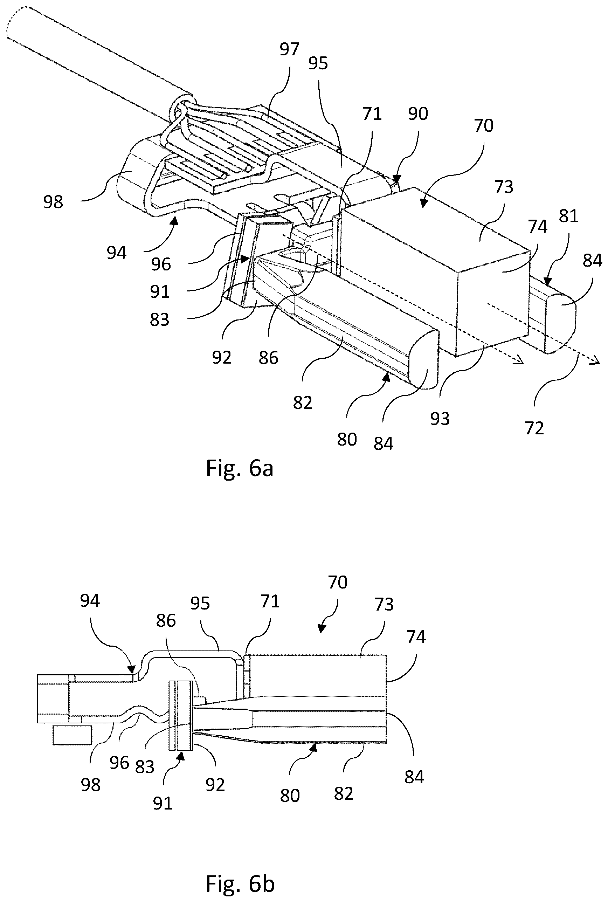

Turning to Figs. 6A-6B showing the tip part 50 without the tip housing 60, the tip part 50 further comprises an electrical circuit 94 provided on a flexible circuit board (FPC). The electrical circuit 94 comprises a folded main circuit portion 98, a first circuit portion 95 in electrical communication with the image sensor 71 and the main circuit portion 98, and a second circuit portion 96 in electrical communication with the first and second light source 90, 91 and the main circuit portion 98. The electrical circuit 94 is configured for transmitting an image signal generated by the image sensor 71 indicative of the view in the optical direction 72 to a circuit (not shown) of the handle 20 of the endoscope 1 via several cables 97 connected to the main circuit portion 98 as best seen in Fig. 6a. The first and the second circuit portions 95, 96 are flexible circuit structures and comprise a slight bend which allow, prior to fixing in the tip housing 60, the imaging subassembly 70 and light sources 90, 91 to move along the longitudinal direction 52, which in this case coincides with the optical direction 72, relative to each other via the flexing of first and second circuit portions 95, 96. The electrical circuit 94 is positioned distally relative to the plug (not shown) and is accommodated in the sealed interior space of the tip housing 60.

A surface normal 93 of each light-emitting surface 92 is preferably oriented in parallel to the optical direction 72 of the image sensor 71 so that light is emitted from the light sources 90, 91 in the same direction as the view of the image sensor 71. The light guide component 80 is arranged so that the imaging subassembly 70 occupies the gap between the light guides 81, 82 and so that the light sources 90, 91 are positioned proximally, i.e. behind, relative to the image sensor 71. This allows a more compact configuration of the imaging subassembly 70 and the light sources 90, 91.

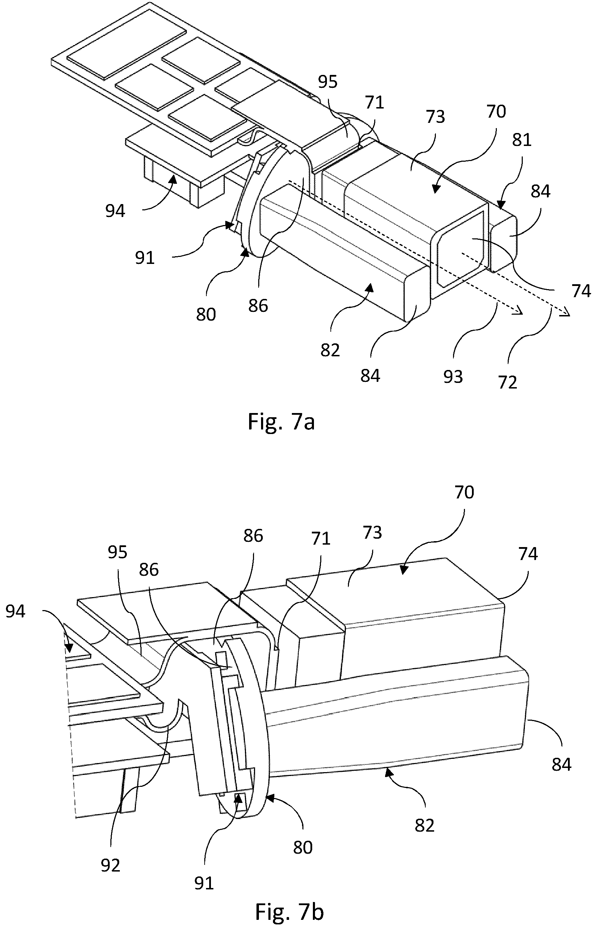

Turning to Fig. 7a-7b, a tip part 50 with a second embodiment of a light guide component 80 is shown without a tip housing and without cables. This second embodiment differs from the previously shown light guide component in that the light guide component comprises a rail 89 at each light entry surface 83, in that the crossmember 85 is plate shaped, and in that each light guide 81, 82 is not asymmetric in a corresponding third plane as described for the first embodiment. As best seen in Fig. 7b, the rails 89 partially surround the respective light entry surface 83 and are configured to retain and provide a seat for the respective light source 90, 91 adhered to the respective light entry surface. The plate-shaped crossmember 85 provides a barrier with only a small gap between the crossmember 85 and an interior surface of the tip housing. Thus, the crossmember 85 prevents sealing adhesive applied proximal to the crossmember 85 from passing the crossmember 85, which could negatively affect the function of the light guides 81, 82.

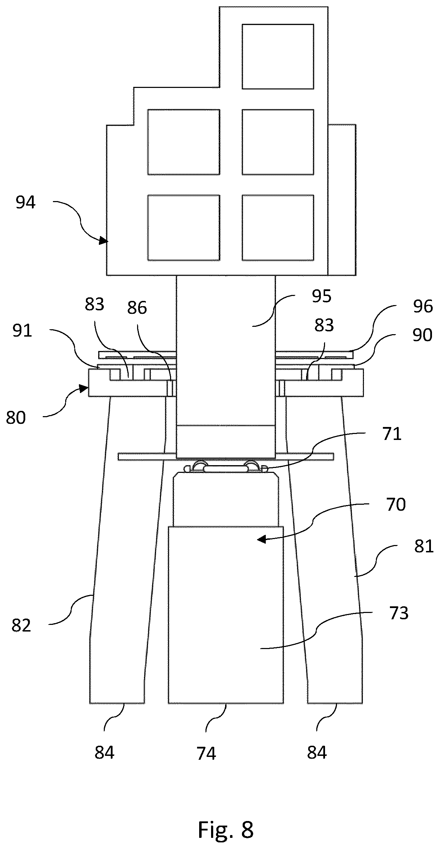

Turning to Fig. 8, a tip part 50 with a third embodiment of a light guide component 80 is shown without a tip housing and without cables. This third embodiment differs from the second embodiment in that the longitudinal central centre line is not straight but rather follows an angled course and comprises an initial outwardly angled part at the light entry surface and a final straight part.

Assembly of the tip part 50 is performed as follows. The tip housing 60, the plug (not shown), the light guide component 80, and the electrical circuit 94 are provided as separately manufactured parts. The tip housing 60, the plug and the light guide component 80 are injection-moulded polymer parts. The electrical circuit 94 are mounted on a frame part (not shown) and has the imaging subassembly 70 and the light sources 90, 91 pre-mounted on the respective circuit portions 95, 96. The light guide component 80 can be any one of the three described embodiments.

The light-emitting surface 92 of first light source 90 is then adhered directly to the light entry surface 83 of the first light guide 81 and the light-emitting surface 92 of the second light source 91 is adhered directly to the light entry surface 83 of the second light guide 82. This ensures that light emitted from the light sources is received by the respective light guide and transmitted out via the respective light exit surface 84, and that the electrical circuit 94, imaging subassembly 70, the light sources 90, 91, and the light guide component 80 can be handled as a main subassembly, e.g. as shown in Figs 4a-4b, Figs. 6a-6b, or Fig. 8.

The main subassembly is then positioned in a jig (not shown) that allows only relative translational adjustment of the light guide component 80 relative to the imaging subassembly 70. The distal end 74 of the lens barrel 73 is then adjusted to a predetermined position relative to the light exit surfaces 84 of the light guide component 80 via flexing of the first and second circuit portions 95, 96. This predetermined position corresponds to the desired position of these components in the tip housing 60 and is in this case that the distal end 74 of the lens barrel 73 is positioned in the same plane as the light exit surfaces 84, as can best be seen in Figs. 6b and 8. The light guide component 80 and the imaging subassembly 70 are then fixed to the frame part to fix their relative position.

This main subassembly is then inserted through the proximal opening 63 of the tip housing 60 into the interior space 62 so that the optical direction 72 of the image sensor 71 extends through the window 65, and so that the light exit surface 84 of the first and second light guides 81, 82 is oriented towards the interior surface 66 of the window 65. The main subassembly is pushed into the interior space 62 until the light exit surfaces 84 and the distal end 74 of the lens barrel 73 abut the interior surface 66 of the window 65 simultaneously due to the prior relative adjustment. The main subassembly is then adhered to the interior surface of the tip housing 60.

The plug (not shown) is then inserted into and closes off the proximal opening 63 while allowing the cables 97 to pass therethrough to the handle 20. An adhesive is applied between the plug and an interior housing surface to fluid and gas seal the interior space 62. The tip part 50 can then be assembled with the bending section 40, the insertion tube 30, and the handle 20, to form the endoscope shown in Fig. 1.

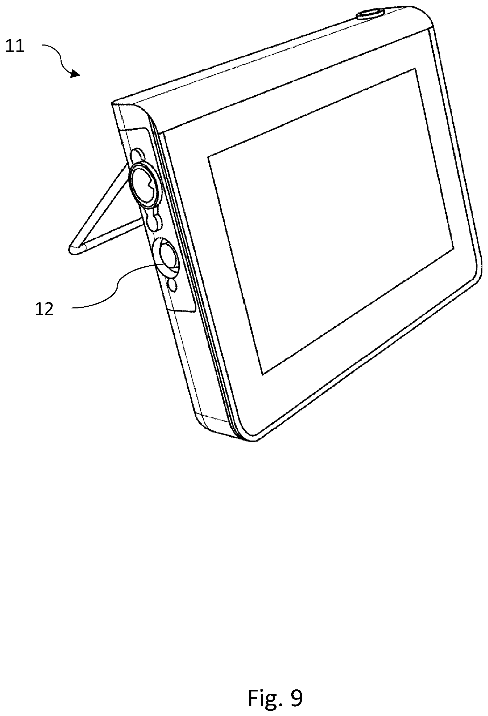

In Fig. 9, a monitor 11 is shown. The monitor 11 comprises a cable socket 12 to which a monitor cable 13 of the endoscope 1 shown in Fig. 1 can be connected to establish signal communication between the image sensor of the endoscope 1 and the monitor 11 via a circuit of the handle 20 connected to the electrical circuit 90 of the tip housing 50 via the cables 97. The monitor 11 display images and/or video captured by the image sensor of the endoscope 1, thus allowing an operator to see through the image sensor of the endoscope 1.

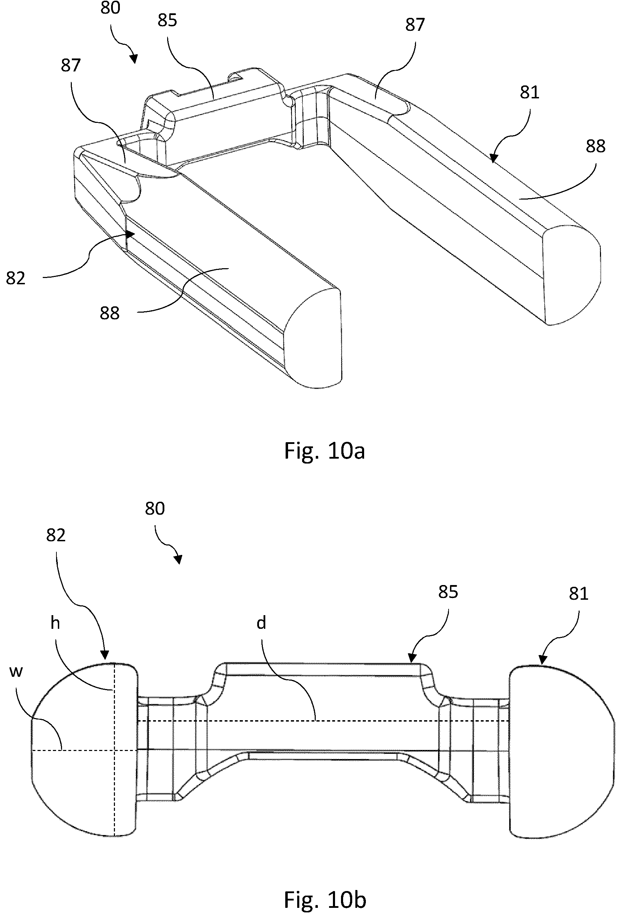

As best seen in Figs. 10a-10b, each light guide 81, 82 comprises a tapering transition portion 87, 82a including the respective light entry surface 83 and a straight portion 82a, 82b directly extending from the transition portion 87 to the respective light exit surface 84. As shown in Fig. 10b, the straight portion 81b, 82b of each light guide 81, 82 has a width w and a height h. Further, the light guides 81, 82 are spaced apart transversely to the longitudinal axis 52 by a distance d. The width w is in the range of 0.35 - 0.55 mm, preferably in the range of 0.4 - 0.5 mm. The height h is in the range of 0.6 - 0.8 mm, preferably in the range of 0. 65 - 0.75 mm. The distance d is in the range of 1.3 - 1.8 mm, preferably in the range of 1.4- 1.7.

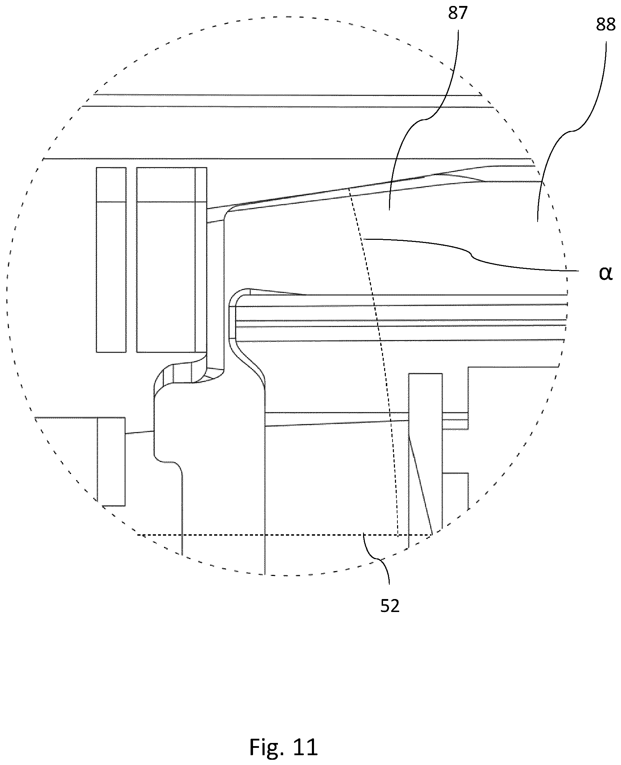

As can be seen on Fig. 11, the transition portion 87 of each light guide 81, 82 tapers from the respective light entry surface 83 to the respective straight portion 88 with a taper angle a with respect to the longitudinal axis 52. The taper angle a is in the range 1 - 12 degrees, preferably 5 - 10 degrees, more preferably 7 - 10 degrees.

### LIST OF REFERENCES

The following is a list of reference numerals used throughout this disclosure. In case of any doubt, the reference numerals of the following list apply.
- 1: endoscope
- 11: monitor
- 12: cable socket
- 13: monitor cable
- 20: handle
- 21: control lever
- 22: handle housing
- 30: insertion tube
- 31: exterior tubular surface
- 32: working channel
- 40: bending section
- 41: proximal end
- 42: sleeve
- 43: segment
- 44: hinge
- 50: tip part
- 51: exterior surface
- 52: longitudinal axis
- 60: housing
- 61: distal end face
- 62: interior space
- 63: proximal opening
- 64: exterior housing surface
- 65: window
- 66: interior window surface
- 67: exterior window surface
- 68: circumferential wall
- 69: distal end wall
- 70: imaging subassembly
- 71: image sensor
- 72: optical direction
- 73: lens barrel
- 74: distal end
- 80: light guide component
- 80a: first plane
- 80b: second plane
- 81: first light guide
- 82: second light guide
- 83: light entry surface
- 84: light exit surface
- 85: circumferential surface
- 86: crossmember
- 87: transition portion
- 88: straight portion
- 89: rail
- a: taper angle
- 90: first light source
- 91: second light source
- 92: light-emitting surface
- 93: central illumination direction
- 94: electrical circuit
- 95: first circuit portion
- 96: second circuit portion
- 97: cable
- 98: main circuit portion

## Claims

1. An endoscope tip part (50) for an endoscope (1) for visually inspecting inaccessible places, such as human body cavities, the endoscope tip part having an exterior surface (51) and extending along a longitudinal axis and comprising:
- a tip housing (60) extending along the longitudinal axis and at least partially enclosing a sealed interior space (62), the tip housing including:
∘ an exterior housing surface (64) for forming a first part of the exterior surface of the endoscope tip part, and
∘ a transparent portion (65), such as a window or a front lens, having an interior surface (66) facing the sealed interior space of the endoscope tip part and an exterior surface (67) forming a second part of the exterior surface of the endoscope tip part;
- an imaging subassembly (70) including an image sensor (71) viewing in an optical direction (72) through the transparent portion of the tip housing;
- a light guide component (80) comprising:
∘ a first and a second light guide (81, 82) being spaced apart by a gap and extending along a respective longitudinal centre line, wherein each light guide has a proximal end, a distal end, a light entry surface (83) at the proximal end, a light exit surface (84) at the distal end, and a circumferential surface (85) extending from the light entry surface to the light exit surface around the respective longitudinal centre line, and
∘ a crossmember (86) extending transversely from the first light guide to the second light guide so as to form a unitary rigid light guide component,
wherein the light guides are configured for propagating light received through the respective light entry surface out through the respective light exit surface and the transparent portion of the tip housing ;
- a first light source (90) having a light-emitting surface (92) and being configured to emit light from the light-emitting surface in a central illumination direction to be received by the light entry surface of the first light guide;
- a second light source (91) having a light-emitting surface (92) and being configured to emit light from the light-emitting surface in a central illumination direction to the light entry surface of the second light guide; and
- an electrical circuit (94) comprising a first circuit portion (95) in electrical communication with the image sensor, the electrical circuit being configured for transmitting an image signal generated by the image sensor indicative of the view in the optical direction;
wherein the first light source, the second light source, the image sensor, and the electrical circuit are accommodated in the sealed interior space of the tip housing,
**characterised in that** the light-emitting surface of the first light source is adhered to the light entry surface of the first light guide of the light guide component by an adhesive, and wherein the light-emitting surface of the second light source is adhered to the light entry surface of the second light guide of the light guide component by an adhesive.

2. An endoscope tip part according to claim 1, wherein the first and second light guides extend side-by-side on opposite sides of the imaging subassembly.

3. An endoscope tip part according to any one of the previous claims, wherein the light guide component is formed separately from the tip housing, and wherein the first light guide exit and/or the second light guide exit is/are oriented towards the interior surface of the transparent portion.

4. An endoscope tip part according to any one of the previous claims, wherein the tip housing comprises a distal end face (61) and a proximal opening (63) providing access to the sealed interior space, wherein the distal end face is positioned opposite the proximal opening, and wherein the distal end face comprises at least part of the exterior surface of the transparent portion.

5. An endoscope tip part according to any one of the previous claims, wherein the first and/or the second light source(s) is/are at least partially overlapping the imaging subassembly when viewed in a cross-section perpendicular to the longitudinal axis.

6. An endoscope tip part according to any one of the previous claims, wherein the first circuit portion is a flexible circuit structure.

7. An endoscope tip part according to any one of the previous claims, wherein the first and/or the second light guide consist(s) essentially of a transparent material with a first refractive index.

8. An endoscope tip part according to any one of the previous claims, wherein the crossmember extends transversely from the proximal end of the first light guide to the proximal end of the second light guide.

9. An endoscope tip part according to any one of the previous claims, wherein the light guide component is formed monolithically.

10. An endoscope tip part according to any one of the previous claims, wherein the light entry surfaces of the first and second light guides are planar, and/or
wherein light exit surfaces of the first and second light guides are planar.

11. An endoscope (1) for visually inspecting inaccessible places such as human body cavities, comprising:
- a handle (20) for gripping by an operator and comprising a control device (21);
- an endoscope tip part (50) according to any one of the claims 1-10;
- an insertion tube (30) for insertion into a patient, the insertion tube extending from the handle to the endoscope tip part and comprising a bending section (40);
- one or more cables (97) running through the insertion tube and electrically connecting the electrical circuit of the endoscope tip part with the handle; and
- at least one steering wire connecting the handle with a distal end of the bending section so that manipulation of the control device causes bending of the bending section.

12. An endoscope system for visually inspecting inaccessible places, such as human body cavities, the endoscope system comprising a monitor (11), and an endoscope (1) according to claim 11 or an endoscope (1) comprising an endoscope tip part (50) according to any one of claims 1-10, wherein the endoscope is connectable to the monitor, and the monitor is configured for displaying an image captured by the image sensor of the endoscope tip part.

13. A method for assembling an endoscope tip part (50), the endoscope tip part extending along a longitudinal axis, the method comprising the steps of:
- providing:
∘ a tip housing (60) extending along the longitudinal axis and at least partially enclosing an interior space (62), the tip housing including:
▪ a proximal opening (63) providing access to the interior space,
▪ an exterior housing surface (64) for forming an exterior surface of the endoscope tip part, and
▪ a transparent portion (65), such as a window or a front lens, having an interior surface (66) and an exterior surface (67), the interior surface facing the interior space of the endoscope tip part and the exterior surface facing the exterior of the endoscope tip part,
∘ an imaging subassembly (70) including an image sensor (71) viewing in an optical direction (72),
∘ a light guide component (80) comprising:
▪ a first and a second light guide (81, 82) being spaced apart by a gap and extending along a respective longitudinal centre line, wherein each light guide has a proximal end, a distal end, a light entry surface (83) at the proximal end, a light exit surface (84) at the distal end, and a circumferential surface (85) extending from the light entry surface to the light exit surface around the respective longitudinal centre line, and
▪ a crossmember (86) extending transversely from the first light guide to the second light guide so as to form a unitary rigid light guide component,
wherein the light guides are configured for propagating light received through the respective light entry surface out through the respective light exit surface and the transparent portion of the tip housing,
∘ a first light source (90) having a light-emitting surface (92) and being configured to emit light from the light-emitting surface in a central illumination direction,
∘ a second light source (91) having a light-emitting surface (92) and being configured to emit light from the light-emitting surface in a central illumination direction, and
∘ an electrical circuit (94) comprising a first circuit portion (95) in electrical communication with the image sensor and a second circuit portion (96) in electrical communication with the light sources, the electrical circuit being configured for transmitting an image signal generated by image sensor indicative of the view in the optical direction, wherein at least one of the first and second circuit portions is flexible so the image sensor and light source(s) are movable relative to each other;
- adhering the first and second light sources to the light guide component, so that light emitted by the first light source is received by the light entry surface of the first light guide, and so that light emitted by the second light source is received by the light entry surface of the second light guide and so that the imaging subassembly, the light guide component, and the electrical circuit form a main subassembly;
- inserting the main subassembly through the proximal opening of the tip housing into the interior space so that the optical direction of the image sensor extends through the transparent portion, and so that surface normals of the light exit surface of the light guides are at least partially oriented towards the interior surface of the transparent portion; and
- fixing the main subassembly to the tip housing and sealing the proximal opening to seal the interior space.

14. A method according to claim 13, further comprising a step of:
- arranging the main subassembly in a jig, adjusting the light guide component relative to the imaging subassembly to a predetermined relative position along the longitudinal direction via flexing of the first and/or second circuit portion, and subsequently fixing the light guide component relative to the imaging subassembly,
wherein the step of inserting the main subassembly comprises inserting the main subassembly along the longitudinal direction until a distal end of the imaging subassembly abuts an interior distal portion of the tip housing, and until a distal end of the light guide component abuts an interior distal portion of the tip housing.

## Patentansprüche

1. Endoskopspitzenteil (50) für ein Endoskop (1) zur visuellen Inspektion unzugänglicher Stellen, wie beispielsweise menschlicher Körperhöhlen, wobei das Endoskopspitzenteil eine Außenfläche (51) aufweist und sich entlang einer Längsachse erstreckt, und umfasst:
- ein Spitzengehäuse (60), das sich entlang der Längsachse erstreckt und mindestens teilweise einen abgedichteten Innenraum (62) umschließt, das Spitzengehäuse einschließend:
∘ eine Gehäuseaußenfläche (64) zur Bildung eines ersten Teils der Außenfläche des Endoskopspitzenteils, und
∘ einen transparenten Abschnitt (65), wie beispielsweise ein Fenster oder eine Frontlinse, der eine Innenfläche (66), die dem abgedichteten Innenraum des Endoskopspitzenteils zugewandt ist, und eine Außenfläche (67), die einen zweiten Teil der Außenfläche des Endoskopspitzenteils bildet, aufweist;
- eine Bildgebungsbaugruppe (70), die einen Bildsensor (71) einschließt, der in einer optischen Richtung (72) durch den transparenten Abschnitt des Spitzengehäuses blickt;
- eine Lichtleiterkomponente (80), umfassend:
∘ einen ersten und einen zweiten Lichtleiter (81, 82), die durch einen Spalt voneinander beabstandet sind und sich entlang einer jeweiligen Längsmittellinie erstrecken, wobei jeder Lichtleiter ein proximales Ende, ein distales Ende, eine Lichteintrittsfläche (83) am proximalen Ende, eine Lichtaustrittsfläche (84) am distalen Ende und eine Umfangsfläche (85), die sich von der Lichteintrittsfläche zu der Lichtaustrittsfläche um die jeweilige Längsmittellinie herum erstreckt, und
∘ einen Querträger (86), der sich quer von dem ersten Lichtleiter zu dem zweiten Lichtleiter erstreckt, um so eine einheitliche, starre Lichtleiterkomponente zu bilden,
wobei die Lichtleiter zum Ausbreiten von durch die jeweilige Lichteintrittsfläche empfangenem Licht durch die jeweilige Lichtaustrittsfläche und den transparenten Abschnitt des Spitzengehäuses ausgebildet sind;
- eine erste Lichtquelle (90), die eine lichtemittierende Oberfläche (92) aufweist und dazu ausgebildet ist, Licht von der lichtemittierenden Oberfläche in einer zentralen Beleuchtungsrichtung zu emittieren, das von der Lichteintrittsfläche des ersten Lichtleiters empfangen werden soll;
- eine zweite Lichtquelle (91), die eine lichtemittierende Oberfläche (92) aufweist und dazu ausgebildet ist, Licht von der lichtemittierenden Oberfläche in einer zentralen Beleuchtungsrichtung auf die Lichteintrittsfläche des zweiten Lichtleiters zu emittieren; und
- eine elektrische Schaltung (94), die einen ersten Schaltungsabschnitt (95) umfasst, der in elektrischer Verbindung mit dem Bildsensor steht, wobei die elektrische Schaltung zum Übertragen eines von dem Bildsensor erzeugten Bildsignals, das den Blick in der optischen Richtung angibt, ausgebildet ist;
wobei die erste Lichtquelle, die zweite Lichtquelle, der Bildsensor und die elektrische Schaltung in dem abgedichteten Innenraum des Spitzengehäuses untergebracht sind, **dadurch gekennzeichnet, dass** die lichtemittierende Oberfläche der ersten Lichtquelle mittels eines Klebstoffs an der Lichteintrittsfläche des ersten Lichtleiters der Lichtleiterkomponente angeklebt ist, und wobei die lichtemittierende Oberfläche der zweiten Lichtquelle mittels eines Klebstoffs an der Lichteintrittsfläche des zweiten Lichtleiters der Lichtleiterkomponente angeklebt ist.

2. Endoskopspitzenteil nach Anspruch 1, wobei sich der erste und der zweite Lichtleiter nebeneinander auf gegenüberliegenden Seiten der Bildgebungsbaugruppe erstrecken.

3. Endoskopspitzenteil nach einem der vorstehenden Ansprüche, wobei die Lichtleiterkomponente getrennt von dem Spitzengehäuse gebildet ist, und wobei der erste Lichtleiteraustritt und/oder der zweite Lichtleiteraustritt in Richtung der Innenfläche des transparenten Abschnitts ausgerichtet ist/sind.

4. Endoskopspitzenteil nach einem der vorstehenden Ansprüche, wobei das Spitzengehäuse eine distale Endfläche (61) und eine proximale Öffnung (63), die Zugang zu dem abgedichteten Innenraum bereitstellt, umfasst, wobei die distale Endfläche gegenüber der proximalen Öffnung positioniert ist, und wobei die distale Endfläche mindestens einen Teil der Außenfläche des transparenten Abschnitts umfasst.

5. Endoskopspitzenteil nach einem der vorstehenden Ansprüche, wobei sich die erste und/oder die zweite Lichtquelle(n) mindestens teilweise mit der Bildgebungsbaugruppe überlappen, wenn im Querschnitt senkrecht zur Längsachse betrachtet.

6. Endoskopspitzenteil nach einem der vorstehenden Ansprüche, wobei der erste Schaltungsabschnitt eine flexible Schaltungsstruktur ist.

7. Endoskopspitzenteil nach einem der vorstehenden Ansprüche, wobei der erste und/oder der zweite Lichtleiter im Wesentlichen aus einem transparenten Material mit einem ersten Brechungsindex besteht/bestehen.

8. Endoskopspitzenteil nach einem der vorstehenden Ansprüche, wobei sich der Querträger quer von dem proximalen Ende des ersten Lichtleiters zu dem proximalen Ende des zweiten Lichtleiters erstreckt.

9. Endoskopspitzenteil nach einem der vorstehenden Ansprüche, wobei die Lichtleiterkomponente monolithisch gebildet ist.

10. Endoskopspitzenteil nach einem der vorstehenden Ansprüche, wobei die Lichteintrittsflächen des ersten und des zweiten Lichtleiters eben sind, und/oder wobei die Lichtaustrittsflächen des ersten und des zweiten Lichtleiters eben sind.

11. Endoskop (1) zur visuellen Inspektion unzugänglicher Stellen, wie beispielsweise menschlicher Körperhöhlen, umfassend:
- einen Griff (20) zum Ergreifen durch einen Bediener und umfassend eine Steuervorrichtung (21);
- ein Endoskopspitzenteil (50) nach einem der Ansprüche 1-10;
- einen Einführschlauch (30) zum Einführen in einen Patienten, wobei sich der Einführschlauch von dem Griff zu dem Endoskopspitzenteil erstreckt und einen Biegeabschnitt (40) umfasst;
- ein oder mehrere Kabel (97), die durch den Einführschlauch verlaufen und die elektrische Schaltung des Endoskopspitzenteils elektrisch mit dem Griff verbinden; und
- mindestens einen Lenkdraht, der den Griff mit einem distalen Ende des Biegeabschnitts verbindet, sodass eine Betätigung der Steuervorrichtung eine Biegung des Biegeabschnitts bewirkt.

12. Endoskopsystem zur visuellen Inspektion unzugänglicher Stellen, wie beispielsweise menschlicher Körperhöhlen, das Endoskopsystem umfassend einen Monitor (11) und ein Endoskop (1) nach Anspruch 11 oder ein Endoskop (1), das ein Endoskopspitzenteil (50) nach einem der Ansprüche 1-10 umfasst, wobei das Endoskop mit dem Monitor verbindbar ist, und der Monitor zum Anzeigen eines von dem Bildsensor des Endoskopspitzenteils aufgenommenen Bildes ausgebildet ist.

13. Verfahren zum Zusammenbau eines Endoskopspitzenteils (50), wobei sich das Endoskopspitzenteil entlang einer Längsachse erstreckt, das Verfahren umfassend die Schritte von:
- Bereitstellen:
∘ eines Spitzengehäuses (60), das sich entlang der Längsachse erstreckt und mindestens teilweise einen Innenraum (62) umschließt, das Spitzengehäuse einschließend:
▪ eine proximale Öffnung (63), die Zugang zu dem Innenraum bereitstellt,
▪ eine Gehäuseaußenfläche (64) zur Bildung einer Außenfläche des Endoskopspitzenteils, und
▪ einen transparenten Abschnitt (65), wie beispielsweise ein Fenster oder eine Frontlinse, der eine Innenfläche (66) und eine Außenfläche (67) aufweist, wobei die Innenfläche dem Innenraum des Endoskopspitzenteils zugewandt ist und die Außenfläche der Außenseite des Endoskopspitzenteils zugewandt ist,
∘ einer Bildgebungsbaugruppe (70), die einen Bildsensor (71) einschließt, der in eine optische Richtung (72) blickt,
∘ einer Lichtleiterkomponente (80), umfassend:
▪ einen ersten und einen zweiten Lichtleiter (81, 82), die durch einen Spalt voneinander beabstandet sind und sich entlang einer jeweiligen Längsmittellinie erstrecken, wobei jeder Lichtleiter ein proximales Ende, ein distales Ende, eine Lichteintrittsfläche (83) am proximalen Ende, eine Lichtaustrittsfläche (84) am distalen Ende und eine Umfangsfläche (85), die sich von der Lichteintrittsfläche zu der Lichtaustrittsfläche um die jeweilige Längsmittellinie herum erstreckt, und
▪ einen Querträger (86), der sich quer von dem ersten Lichtleiter zu dem zweiten Lichtleiter erstreckt, um so eine einheitliche, starre Lichtleiterkomponente zu bilden,
wobei die Lichtleiter zum Ausbreiten von durch die jeweilige Lichteintrittsfläche empfangenem Licht durch die jeweilige Lichtaustrittsfläche und den transparenten Abschnitt des Spitzengehäuses ausgebildet sind,
∘ einer ersten Lichtquelle (90), die eine lichtemittierende Oberfläche (92) aufweist und dazu ausgebildet ist, Licht von der lichtemittierenden Oberfläche in einer zentralen Beleuchtungsrichtung zu emittieren,
∘ einer zweiten Lichtquelle (91), die eine lichtemittierende Oberfläche (92) aufweist und dazu ausgebildet ist, Licht von der lichtemittierenden Oberfläche in einer zentralen Beleuchtungsrichtung zu emittieren, und
∘ einer elektrischen Schaltung (94), die einen ersten Schaltungsabschnitt (95) in elektrischer Verbindung mit dem Bildsensor und einen zweiten Schaltungsabschnitt (96) in elektrischer Verbindung mit den Lichtquellen umfasst,
wobei die elektrische Schaltung zum Übertragen eines von dem Bildsensor erzeugten Bildsignals, das den Blick in der optischen Richtung angibt, ausgebildet ist, wobei mindestens einer von dem ersten und dem zweiten Schaltungsabschnitt flexibel ist, sodass der Bildsensor und die Lichtquelle(n) relativ zueinander beweglich sind;
- Ankleben der ersten und der zweiten Lichtquelle an der Lichtleiterkomponente, sodass von der ersten Lichtquelle emittiertes Licht von der Lichteintrittsfläche des ersten Lichtleiters empfangen wird und sodass von der zweiten Lichtquelle emittiertes Licht von der Lichteintrittsfläche des zweiten Lichtleiters empfangen wird, und sodass die Bildgebungsbaugruppe, die Lichtleiterkomponente und die elektrische Schaltung eine Hauptbaugruppe bilden;
- Einführen der Hauptbaugruppe durch die proximale Öffnung des Spitzengehäuses in den Innenraum, sodass sich die optische Richtung des Bildsensors durch den transparenten Abschnitt erstreckt, und sodass Oberflächennormalen der Lichtaustrittsfläche der Lichtleiter mindestens teilweise in Richtung der Innenfläche des transparenten Abschnitts ausgerichtet sind; und
- Fixieren der Hauptbaugruppe an dem Spitzengehäuse und Abdichten der proximalen Öffnung, um den Innenraum abzudichten.

14. Verfahren nach Anspruch 13, weiter umfassend einen Schritt von:
- Anordnen der Hauptbaugruppe in einer Montagevorrichtung, Einstellen der Lichtleiterkomponente relativ zur Bildgebungsbaugruppe auf eine vorbestimmte relative Position entlang der Längsrichtung durch Biegen des ersten und/oder des zweiten Schaltungsabschnitts und nachfolgendes Fixieren der Lichtleiterkomponente relativ zur Bildgebungsbaugruppe,
wobei der Schritt des Einführens der Hauptbaugruppe ein Einführen der Hauptbaugruppe in Längsrichtung umfasst, bis ein distales Ende der Bildgebungsbaugruppe an einem inneren distalen Abschnitt des Spitzengehäuses anliegt und bis ein distales Ende der Lichtleiterkomponente an einem inneren distalen Abschnitt des Spitzengehäuses anliegt.

## Revendications

1. Partie (50) de pointe d'endoscope destinée à un endoscope (1) pour inspecter visuellement des endroits inaccessibles, tels que des cavités du corps humain, la partie de pointe d'endoscope présentant une surface extérieure (51) et s'étendant selon un axe longitudinal, et comprenant :
- un logement (60) de pointe s'étendant le long de l'axe longitudinal et enfermant au moins partiellement un espace intérieur (62) isolé, le logement de pointe incluant :
∘ une surface extérieure (64) de logement pour former une première partie de la surface extérieure de la partie de pointe d'endoscope, et
∘ une portion transparente (65), telle qu'une fenêtre ou une lentille avant, présentant une surface intérieure (66) tournée vers l'espace intérieur isolé de la partie de pointe d'endoscope et une surface extérieure (67) formant une seconde partie de la surface extérieure de la partie de pointe d'endoscope ;
- un sous-ensemble d'imagerie (70) incluant un capteur (71) d'image dirigé dans une direction optique (72) à travers la portion transparente du logement de pointe ;
- un composant guide (80) de lumière comprenant :
∘ un premier et un second guide (81, 82) de lumière étant séparés par un espace et s'étendant le long d'une ligne médiane longitudinale respective, dans laquelle chaque guide de lumière présente une extrémité proximale, une extrémité distale, une surface d'entrée (83) de lumière à l'extrémité proximale, une surface de sortie (84) de lumière à l'extrémité distale, et une surface circonférentielle (85) s'étendant de la surface d'entrée de lumière à la surface de sortie de lumière autour de la ligne médiane longitudinale respective, et
∘ un élément transversal (86) s'étendant transversalement du premier guide de lumière au second guide de lumière afin de former un composant guide de lumière rigide unitaire,
dans laquelle les guides de lumière sont configurés pour propager la lumière reçue à travers la surface d'entrée de lumière respective vers l'extérieur, à travers la surface de sortie de lumière respective et la portion transparente du logement de pointe ;
- une première source (90) de lumière présentant une surface (92) d'émission de lumière et étant configurée pour émettre de la lumière à partir de la surface d'émission de lumière dans une direction d'éclairage centrale, destinée à être reçue par la surface d'entrée de lumière du premier guide de lumière ;
- une seconde source (91) de lumière présentant une surface (92) d'émission de lumière et étant configurée pour émettre de la lumière de la surface d'émission de lumière, dans une direction d'éclairage centrale, à la surface d'entrée de lumière du second guide de lumière ; et
- un circuit électrique (94) comprenant une première portion (95) de circuit en communication électrique avec le capteur d'image, le circuit électrique étant configuré pour émettre un signal d'image généré par le capteur d'image indiquant la vue dans la direction optique ;
dans laquelle la première source de lumière, la seconde source de lumière, le capteur d'image et le circuit électrique sont logés dans l'espace intérieur isolé du logement de pointe, **caractérisée en ce que** la surface d'émission de lumière de la première source de lumière est collée à la surface d'entrée de lumière du premier guide de lumière du composant guide de lumière par un adhésif, et dans laquelle la surface d'émission de lumière de la seconde source de lumière est collée à la surface d'entrée de lumière du second guide de lumière du composant guide de lumière par un adhésif.

2. Partie de pointe d'endoscope selon la revendication 1, dans laquelle les premier et second guides de lumière s'étendent côte à côte sur des côtés opposés du sous-ensemble d'imagerie.

3. Partie de pointe d'endoscope selon l'une quelconque des revendications précédentes, dans laquelle le composant guide de lumière est formé séparément du logement de pointe, et dans laquelle la première sortie de guide de lumière et/ou la seconde sortie de guide de lumière est/sont orientée(s) vers la surface intérieure de la portion transparente.

4. Partie de pointe d'endoscope selon l'une quelconque des revendications précédentes, dans laquelle le logement de pointe comprend une face (61) d'extrémité distale et une ouverture proximale (63) permettant d'accéder à l'espace intérieur isolé, dans laquelle la face d'extrémité distale est positionnée à l'opposé de l'ouverture proximale, et dans laquelle la face d'extrémité distale comprend au moins une partie de la surface extérieure de la portion transparente.

5. Partie de pointe d'endoscope selon l'une quelconque des revendications précédentes, dans laquelle la première et/ou la seconde sources de lumière chevauchent au moins partiellement le sous-ensemble d'imagerie lorsqu'elles sont vues en coupe transversale perpendiculaire à l'axe longitudinal.

6. Partie de pointe d'endoscope selon l'une quelconque des revendications précédentes, dans laquelle la première portion de circuit est une structure de circuit souple.

7. Partie de pointe d'endoscope selon l'une quelconque des revendications précédentes, dans laquelle le premier et/ou le second guides de lumière consistent essentiellement en un matériau transparent présentant un premier indice de réfraction.

8. Partie de pointe d'endoscope selon l'une quelconque des revendications précédentes, dans laquelle l'élément transversal s'étend transversalement de l'extrémité proximale du premier guide de lumière à l'extrémité proximale du second guide de lumière.

9. Partie de pointe d'endoscope selon l'une quelconque des revendications précédentes, dans laquelle le composant guide de lumière est formé monolithiquement.

10. Partie de pointe d'endoscope selon l'une quelconque des revendications précédentes, dans laquelle les surfaces d'entrée de lumière des premier et second guides de lumière sont planes, et/ou dans laquelle les surfaces de sortie de lumière des premier et second guides de lumière sont planes.

11. Endoscope (1) pour inspecter visuellement des endroits inaccessibles tels que des cavités du corps humain, comprenant :
- un manche (20) destiné à être saisi par un opérateur et comprenant un dispositif de commande (21) ;
- une partie (50) de pointe d'endoscope selon l'une quelconque des revendications 1 à 10 ;
- un tube d'insertion (30) pour insertion dans un patient, le tube d'insertion s'étendant du manche à la partie de pointe d'endoscope et comprenant une section flexible (40) ;
- un ou plusieurs câbles (97) passant à travers le tube d'insertion et connectant électriquement le circuit électrique de la partie de pointe d'endoscope au manche ; et
- au moins un fil de guidage reliant le manche à une extrémité distale de la section flexible, de sorte que la manipulation du dispositif de commande provoque la flexion de la section flexible.

12. Système d'endoscope pour inspecter visuellement des endroits inaccessibles, tels que des cavités du corps humain, le système d'endoscope comprenant un moniteur (11), et un endoscope (1) selon la revendication 11 ou un endoscope (1) comprenant une partie (50) de pointe d'endoscope selon l'une quelconque des revendications 1 à 10, dans lequel l'endoscope est connectable au moniteur, et le moniteur est configuré pour afficher une image capturée par le capteur d'image de la partie de pointe d'endoscope.

13. Procédé d'assemblage d'une partie (50) de pointe d'endoscope, la partie de pointe d'endoscope s'étendant le long d'un axe longitudinal, le procédé comprenant les étapes de :
- fourniture de :
∘ un logement (60) de pointe s'étendant le long de l'axe longitudinal et enfermant au moins partiellement un espace intérieur (62), le logement de pointe incluant :
▪ une ouverture proximale (63) permettant l'accès à l'espace intérieur,
▪ une surface extérieure (64) de logement destinée à former une surface extérieure de la partie de pointe d'endoscope, et
▪ une portion transparente (65), telle qu'une fenêtre ou une lentille avant, présentant une surface intérieure (66) et une surface extérieure (67), la surface intérieure étant tournée vers l'espace intérieur de la partie de pointe d'endoscope et la surface extérieure étant tournée vers l'extérieur de la partie de pointe d'endoscope,
∘ un sous-ensemble d'imagerie (70) incluant un capteur (71) d'image dirigé dans une direction optique (72),
∘ un composant guide (80) de lumière comprenant :
▪ un premier et un second guide (81, 82) de lumière étant séparés par un espace et s'étendant le long d'une ligne médiane longitudinale respective, dans laquelle chaque guide de lumière présente une extrémité proximale, une extrémité distale, une surface d'entrée (83) de lumière à l'extrémité proximale, une surface de sortie (84) de lumière à l'extrémité distale, et une surface circonférentielle (85) s'étendant de la surface d'entrée de lumière à la surface de sortie de lumière autour de la ligne médiane longitudinale respective, et
▪ un élément transversal (86) s'étendant transversalement du premier guide de lumière au second guide de lumière afin de former un composant guide de lumière rigide unitaire,
dans lequel les guides de lumière sont configurés pour propager la lumière reçue à travers la surface d'entrée de lumière respective vers l'extérieur, à travers la surface de sortie de lumière respective et la portion transparente du logement de pointe,
∘ une première source (90) de lumière présentant une surface (92) d'émission de lumière et étant configurée pour émettre de la lumière à partir de la surface d'émission de lumière dans une direction d'éclairage centrale,
∘ une seconde source (91) de lumière présentant une surface (92) d'émission de lumière et étant configurée pour émettre de la lumière à partir de la surface d'émission de lumière dans une direction d'éclairage centrale, et
∘ un circuit électrique (94) comprenant une première portion (95) de circuit en communication électrique avec le capteur d'image et une seconde portion (96) de circuit en communication électrique avec les sources de lumière,
le circuit électrique étant configuré pour émettre un signal d'image généré par un capteur d'image indiquant la vue dans la direction optique, dans lequel au moins l'une des première et seconde portions de circuit est souple de sorte que le capteur d'image et la ou les sources de lumière soient mobiles entre eux ;
- adhérence des première et seconde sources de lumière au composant guide de lumière, de sorte que la lumière émise par la première source de lumière soit reçue par la surface d'entrée de lumière du premier guide de lumière, et de sorte que la lumière émise par la seconde source de lumière soit reçue par la surface d'entrée de lumière du second guide de lumière et de sorte que le sous-ensemble d'imagerie, le composant guide de lumière et le circuit électrique forment un sous-ensemble principal ;
- insertion du sous-ensemble principal à travers l'ouverture proximale du logement de pointe dans l'espace intérieur de sorte que la direction optique du capteur d'image s'étende à travers la portion transparente, et de sorte que les normales de surface de la surface de sortie de lumière des guides de lumière soient au moins partiellement orientées vers la surface intérieure de la portion transparente ; et
- fixation du sous-ensemble principal au logement de pointe et obturation de l'ouverture proximale pour isoler l'espace intérieur.

14. Procédé selon la revendication 13, comprenant en outre une étape de :
- agencement du sous-ensemble principal dans un gabarit, ajustement du composant guide de lumière par rapport au sous-ensemble d'imagerie en une position relative prédéterminée le long de la direction longitudinale en fléchissant la première et/ou la seconde portion de circuit, puis fixation du composant guide de lumière par rapport au sous-ensemble d'imagerie,
dans lequel l'étape d'insertion du sous-ensemble principal comprend l'insertion du sous-ensemble principal le long de la direction longitudinale jusqu'à ce qu'une extrémité distale du sous-ensemble d'imagerie vienne en butée contre une portion distale intérieure du logement de pointe, et jusqu'à ce qu'une extrémité distale du composant guide de lumière vienne en butée contre une portion distale intérieure du logement de pointe.
